# EUROPEAN PATENT APPLICATION

(11) **EP 0 562 624 A2**
(43) Date of publication of application: **29.09.1993**
(21) Application number: 93105044.7
(22) Date of filing: 26.03.1993
(51) Int. Cl.: C12N 9/96, C12N 11/00, C12N 9/00

(54) **Stabilization of functional proteins**

(30) Priority: 26.03.1992 US 858399
(71) Applicant: Modrovich, Ivan E., Camarillo CA 93010 (US)
(72) Inventor: Kwan, Shing Fai, Ventura, California 93304 (US); Bravo-Leerabhandh, Marjorie, Thousand Oaks, California 91320 (US); Hunt, Rebecca Jolene, Carpinteria, California 93013 (US)
(74) Representative: Grünecker, Kinkeldey, Stockmair & Schwanhäusser Anwaltssozietät

(57) **Abstract**

Labile analytes are stabilized by reaction with a biostabilizer in the presence of a condensing or linking agent.

## Description

### Field of the Invention

The present invention is related to the stabilization of labile analytes for use in clinical assay, control and calibration compositions.

### Background of the Invention

The present invention pertains to stabilization of labile proteins which retain their stability in a liquid media for protracted periods of time from 2 to 10°C.

The assignee of the instant invention has, over the years, been concerned with the providing of stable liquid compositions which function as diagnostic reagents as well as controls and calibrators.

The compositions sought to be developed must have a stability for a commercially sufficient period of time. A normal goal is to provide stability of 12 to 18 months at 4 to 10°C, the latter period corresponding to a stability under stress of 3 days at 41°C. The composition having such a stability will survive the period of time from manufacture and storage, shipment to the four corners of the world and storage at destination until time of use.

It has been recognized that labile enzymes, and other functional proteins are relatively unstable and rapidly degrade in the presence of water.

The stabilization of enzymes by immobilization using a variety of agents is described in "Enzyme Stabilization by Immobilization" by Klibanov Analytical Chemistry 93,1-25 1979, and in Chapter 1 of "Protein Function: A Practical Approach" by Creighton, IRL Press, both incorporated herein by reference. Techniques include covalent attachment to water insoluble and water soluble supports, adsorption on various surfaces, covalent cross-linking with bi- (or poly-) functional reagents to produce water-soluble or water-insoluble derivatives, entrapment in polymeric gels and encapsulation in microcapsules or hollow fibers.

U.S. Patent 4,652,524 to Modrovich et al, incorporated herein by reference, describes a method and composition for stabilizing labile enzymes. The method comprises the steps of reacting in a liquid media an enzyme with a polymer having pending anhydride groups capable of covalently bonding to and immobilizing the enzyme. The polymer is a copolymer of ethylene and maleic anhydride in which the functional pendant maleic anhydride groups bond to the pendant groups on the enzyme.

We have been most familiar with the procedure described in the '524 patent.

Stabilization procedures described therein while having broad utility does not work for all enzymes. An example is with glycerol phosphate oxidase (GPO) which will not link to the pendant maleic anhydride functional group. Because the functional group is an anhydride it is highly reactive and this leads to problems in controlling the reaction. All the anhydride groups will, under conditions of reaction, react with enzymes and other agents as well as form bridging cross-links between polymer backbones. In consequence, the reaction is somewhat random leading to, at times, the formation of polymers which are relatively hydrophobic. Hydrophobicity makes it difficult to form highly concentrated solutions in an aqueous media.

In addition the polymer backbone is fairly rigid, which may be the reason why it is not useful for stabilization of some enzymes like glycerol phosphate oxidase.

It would be desirable to provide more utilitarian stabilizing reactants which will stabilize under milder conditions, a wide variety of enzymes and provide hydrophilic products which can be dissolved in an aqueous media at high concentrations. This is the subject of the instant invention.

### Summary of the Invention

The present invention is directed to the stabilization of proteinaceous analytes, particularly labile enzymes to provide water soluble products of protracted activity for use in liquid and lyophilized in vitro diagnostic reagents, controls and calibrators.

According to the present invention the labile analyte stabilization is realized by reacting the labile analyte with a biostabilizer in an aqueous media in the presence of a condensing or linking agent. There is first providing a solution of the labile analyte to be stabilized under suitable refrigerated conditions (greater than 0 and up to about 10°C) to which a solution of a biostabilizer is added slowly at a reduced temperature of greater than 0 and up to about 10°C with mixing. Following this, there is added in a solution of a condensing agent which enhances the formation of covalent bonds between the labile analyte and the biostabilizer. The condensing agent may cause and/or enter into the reaction and become part of the soluble stabilized product.

In the practice of the invention, a weight ratio of labile analyte to biostabilizer of 100:1 or less is required to improve analyte stability. In any event, the amount of biological stabilizer to be utilized, need be no more than that needed to provide the required stability for the enzyme. As compared to the previous technique of covalently bonding the enzymes to pendant maleic anhydride groups, the present invention is more universally applicable and provides a hydrophilic product formed under more gentle reaction conditions, normally amounting to no more allowing the reactants to stand for a few days under refrigeration conditions. Reaction may be enhanced by incubation at elevated temperatures.

### Brief Description of the Drawing

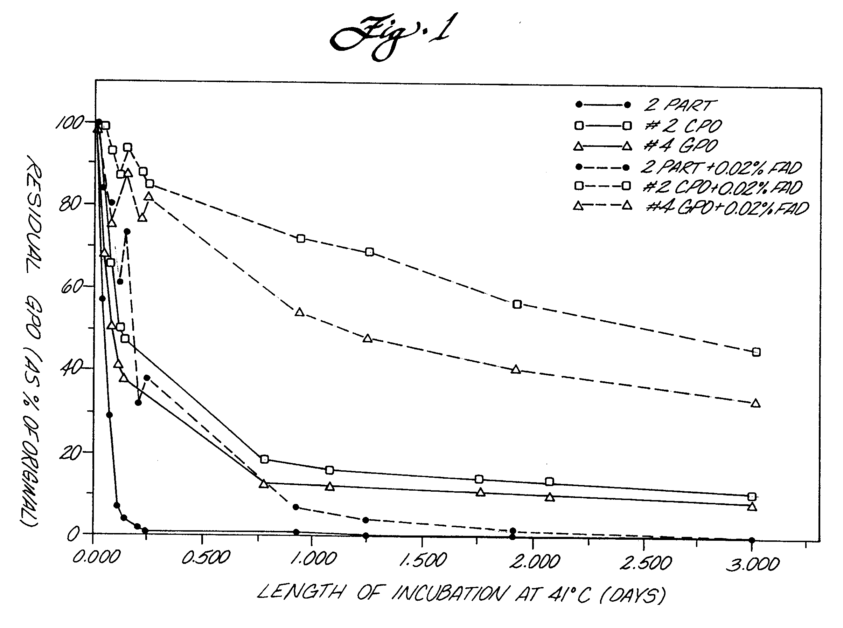

FIG. 1 compares the relative stability of glycerophosphate oxidase (GPO) as a percent of original against incubation time under stress at 41°C.

In the legend, "2 Part" means a two component triglyceride reagent containing GPO; "#2 GPO" and "#4 GPO" means a single reagent using GPO stabilized in accordance with the invention, while flavin adenine dinucleotide ("FAD") means the coenzyme FAD has been added to the composition under evaluation.

The graph confirms the significant stability induced by practice of the instant invention, particularly when combined with the FAD coenzyme.

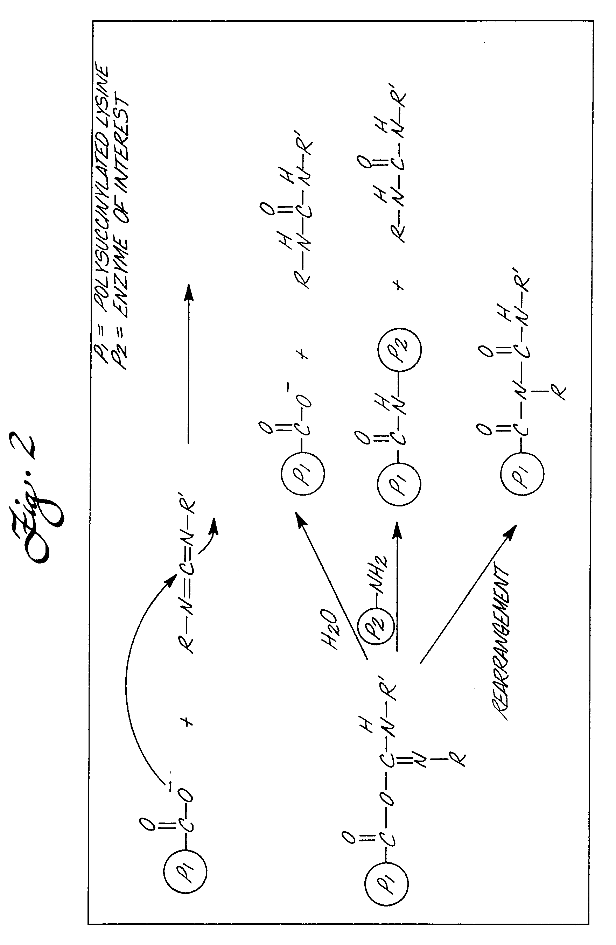

Attached FIG. 2 illustrates, as explained herein, how one method of stabilization of the labile analytes occur; wherein P₁ is the biostabilizer such as polysuccinylated lysine and P₂ is the labile analyte of interest to be stabilized.

### Detailed Description

According to the present invention there is provided labile analytes stabilized by a controlled reaction with biostabilizer in the presence of a condensing agent which has broad utility in terms of the labile analytes which can be stabilized and provides a hydrophilic product of high solubility in water.

In accordance with the invention to an aqueous solution containing the labile analyte to be stabilized there is slowly added, typically under dropwise conditions, a biostabilizer which is reactive with the labile analyte followed by addition of a condensing or linking agent solution which initiates and may enter into the reaction with the biostabilizer and labile analyte. The combination is allowed to react under refrigerated conditions of greater than 0 and up to about 10°C preferably about 4°C and used as such in a reagent, controls or calibrators. Incubation at elevated temperatures may be used to enhance completion of the reaction.

By the expression "biostabilizer" there is meant a biological material as defined herein which will enter into a cross-linking covalent reaction with a labile analyte directly or through a condensing agent to immobilize the analyte in an active form.

By the term "condensing or linking agent" as used herein there is meant a compound which will cause or enter into a covalent cross-linking reaction involving the biostabilizer and labile analyte.

By the term "labile analyte" there is meant a proteinaceous material including enzymes which lose activity and alter their natural state in an aqueous media.

The biostabilizers which are employed to bind to labile analytes are water soluble, hydrophilic compounds which inherently contain or may be modified to contain one or more sites which are reactive with sites on the analyte. The biostabilizers include biopolymers such as polyarginine, poly-dl-lysine, poly-l-lysine, poly-dl-aspartate, poly-l-aspartate, poly-l-glutamic acid, polysuccinylated-lysine, polysacharides and the like and naturally occurring and modified proteins such as gelatin, albumin, collagen, fibrinogen and the like. Polysuccinylated lysine MW 220,000 ("PSL", herein) is presently preferred.

The biostabilizer have well defined molecular weights between about 30,000 and 220,000 are low in cost and have a multitude of functional groups for covalent linkage (cross-linking). The functional groups are in categories comprising N-terminal amino groups, C-terminal carboxyl groups, thiol groups, thioether groups, imidezol groups, guanidino groups, phenolic hydroxyl groups, and indolyl groups. At least one of these functional groups must exist in the labile analyte and the biostabilizer in order for the desired covalent cross-linking to occur.

The condensing agent initiates and may enter into the cross-linking reaction. They are molecules which contain appropriate reactive groups to initiate covalent linkage between the biostabilizer and the labile analyte. The condensing agent may activate and/or enter into the reaction, e.g. serve as a cross-linking group between the biostabilizer and analyte.

The covalent linkage can either be between the same functional groups or different functional groups. There are three types of condensing reagents, namely: homobifunctional, heterobifunctional, and zero-length reagents. There are hundreds of reagents which fit these categories.

In homobifunctional reagents, the functional groups involved in the reaction between the labile analyte and the biostabilizer are the same. Without limitation some are listed below by class.

One class is amine group directed which include:
bis-imidoesters, which readily react with amino groups eliminating an alcohol to form amidines. Examples include diethyl malonimidate, dimethyl succinimidate, dimethyl glutarimidate, methyl acetimidate, dimethyl sebacimidate,diiosvthionyl 3,3'-dithiobispropionimidate and the like.

Also included are bis-succinimidyl derivatives: synthesized by condensing N-hydroxysuccinimide with the corresponding dicarboxylic acids in the presence of dicyclohexylcarbodiimide. They react preferentially with amino groups eliminating N-hydroxysuccinimide as the leaving group. Examples include: bis(sulfosuccinimidyl)-suberate, succinate bis-(N-hydroxysuccinimide ether), bis (sulfo-N-succinimidyl)-oxyl-2-spiro-5'-azelate, 2,2'-dithiobis(succinimidylpropionate), disuccinimidyl tartarate, N,N'-bis(3-succinimidyloxycarbonylpropyl) tartarimide and the like.

Bifunctional aryl halides which react preferentially with amino and tyrosine phenolic groups, and which also react with thiol and imidazolyl groups. The electron withdrawing carboxyl and nitro groups on the benzene ring of such compounds activate the halogen for nucleophilic substitution. Examples include 1,5-difluoro-2,4-dinitrobenzene, 1,5-dichloro-2,4-dinitrobenzene, 1,5-dibromo-2, 4-dinitrobenzene, Bis (3,5-dibromosalicyl) succinate, 4,4'-difluoro-3,3'-dinitrodiphenylsulfone and the like.

Also functional are bifunctional acylating agents which are generally diisocyanates and diisothiocyanates such as 1,6-hexamethylene diisocyanate, xylene diisocyanate, benzidine diisocyanate, diphenylmethane-4,4'-diisocyanate, hexahydrobiphenyl-4,4'diisocyanate, diphenyl-4,4'diisothiocyanato-2,2'-disulfonic acid and the like; bifunctional sulfonyl halides which include phenol, 2,4-disulfonyl chloride, a-naphthol-2,4-disulfonyl chloride, naphthalene-1,5-disulfonyl chloride and the like.

Functional dinitrophenol esters may also be used which include the bis(p-nitrophenyl ester) of carboxylic acids; bis-(p-nitrophenyl)adipate, bis-(p-nitrophenyl) suberate, carbonyl bis (L-methionine p-nitrophenyl) ester and the like.

Biofunctional acylazides may be used which include tartryl diazide, p-bis-(ureido)azidooligopropylazobenzene, trimesyl-tris-B-alanylazide and the like.

Functional dialdehydes include glyoxal, malodialdehyde, succinialdehyde, adipaldehyde, glutaraldehyde, 2-methoxy-2,4-dimethylglutaraldehyde, 3-methylglutaraldehyde and the like.

Functional diketones include 2,5-hexanedione, 3,4-dimethyl-2,5-hexanedione and the like.

Other compounds of this class include benzoquinone, mucobromic acid, mucochloric acid, 2-iminothiolane, ethylchloroformate, erythreitolbiscarbonate, p-nitrophenylchloroformate and the like.

Yet other classes of biological macromolecules are sulfhydryl reagents which consist of derivatives of mercury, maleimide, disulfide, halomethylketone, and other alkylating agents; mercurial reagents which include 3,6-bis(chloromercurimethyl) dioxane, 3,6-bis(nitromercurimethyl) dioxane, 1,4-bis(bromomercuri) butane, and the like; disulfide forming reagents which include 3-oxy-2,2-bis[{((2-((3-carboxy-4-nitrophenyl)dithiol)ethyl)amino)carbonyl]hexanyl]-4,4-dimethyl-oxazolidine, 5,5'-pentamethylenebis(methanethiosulfonate), 12,12'-dodecamethylenebis(methanethiosulfonate), crabescein and the like; bismaleimides which include N,N'methylenebismaleimide, N,N'-trimethylenebismaleimide, Bis(N-maleimido)-1,6-hexane, Bis(N-maleimido-1,8-octane, Bis(N-maleimidomethyl) ether, 2,2-bis(maleimidomethoxy)-propane, and the like.

Alkylating agents which are bio-haloacetyl derivatives may be used which include 1,3-dibromoacetone, N,N'-ethylene-bis(iodoacetamide), N,N'-hexamethylene-bis(iodoacetamide), N,N'-di(bromoacetyl)phenylhydrazine, 1,2-di(bromoacetyl)amino-3-phenyl hydrazine and the like; while di-alkyl halides include a,a'-dibromo-p-xylene sulfonic acid, di(2-chloroethyl)sulfide, tri(2'chloroethyl)amine, N,N-bis(B-bromoethyl)benzylamine, 1,3-bis(2-chloroethyl)-1-nitrosourea and the like; s-triazines include 2,4-dichloro-6-methoxy-s-triazine, 2,4-dichloro-6-amino-s-triazine, 2,4-dichloro-6-(5'sulfonic acid naphthaleneamino)-s-triazine and the like; aziridine reagents include 2,4,6-tri(ethyleneimino)-s-triazine, N,N'-ethyleneiminoyl-1,6-diaminohexane, tri{1-(2-methylanziridenyl)}-phosphine oxide and the like; while functional bis-epoxides include 1,2:3,4-diepoxybutane, 1,2:5,6-diepoxyhexane, bis(2,3-epoxypropyl)ether, 1,4-butadioldiglycidoxyether, 3,4-isopropylidene-1,2:5,6-dianhydro-mannitol and the like.

Other classes are carboxyl directed groups which include 1,1-bis(diazoacetyl)-2-phenylethane bisdiazohexane and the like and phenolate and imidazolyl groups directed, examples of which include p-phenylenediamine, bis-benzidine, 3,3'-dimethoxybenzidine, benzidine-2, 2'-disulfonic acid, 4,4'-diaminodiphenyldisulfide, 4,4'-diaminodiphenylamine, 2,2'-dinitro-4,4'-diaminodiphenyl-disulfide, poly(p-amino-D,L-phenylalanyl-L-leucine), and the like; while guanidino groups directed include p-phenylenediglyoxal and the like.

In contrast to homobifunctional reagents, heterobifunctional reagents contain two dissimilar reactive groups of different specificities. The functional group involved in the cross-linking on the enzyme is different from that on the biostabilizer.

Without limitation they include amino and sulfhydryl directed groups which include N-succinimidyl 3-(2-pyridyldithio)propionate, N-succinimidyl maleimidoacetate, N-succinimidyl 4-(N-maleimidomethyl) cyclohexane-1-carboxylate, N-succinimidyl 4-(p-maleimidophenyl)butyrate, N-sulfosuccinimidyl m-maleimidobenzoate, N-succinimidyliodoacetate, p-nitrophenyl 6-maleimidocaproate p-nitrophenyliodoacetate, 2,4-dinitrophenyl-p-(B-nitrovinyl)benzoate. ethyl iodoacetimidate HC1, 4-maleimidobenzoylchloride, 4-chloroacetylphenylmaleimide, a,a-bis{(p-tolylsulfonyl)methyl}-4- nitroacetophenone, and the like; carboxyl and either sulfhydryl or amino directed groups which include 1-(Aminooxy)-4-{(3-nitro-2-pyridyl)dithio}butane, 1-(aminooxy)-4-{(3-nitro-2-pyridyl)dithio}but-2-ene, and the like.

The third class of condensing reagents is zero-length reagents. These reagents are a special class of compounds. They induce direct joining of two chemical groups of proteins without introducing any additional atoms or molecules. During the condensing reaction, moieties are eliminated from the reactants leaving exposed ends that react together to form covalent bonds.

This differs from the homo- and heterobifunctional reagents as in these reactions there are spacers that are incorporated between the two crosslinked groups.

Examples of zero-length cross-linking reagents include, but are certainly not limited to carboxyl activator groups such as carbodiimides for linking of carboxyl and amino groups and include dihexylcarbodiimide, 1-ethyl-3-(3-dimethylaminopropyl)carbodiimide HC1, 1-ethyl-3-(4-azonia-4,4-dimethypentyl)carbodiimide iodide, N-benzyl-N'-3-dimethylaminopropyl-carbodiimide Hc1 and the like.

Isoxasolium derivatives for linking of carboxyl and amino groups which include N-ethyl-3-phenylisoxazolium-3'-sulfonate, N-ethylbenzisoxazolium tetrafluoroborate and the like; chloroformates for linking of carboxyl and amino groups which include ethylchloroformate, p-nitrophenylchloroformate, 2,4,5-trichlorophenylchloroformate, and the like; carbonylidiimidazoles for linking of carboxyl and amino groups which include 1,1'-carbonyldiimidazole and the like; N-carbalkoxydihydroquinolines for linking of carboxyl and amino groups which include N-(ethoxycarbonyl)-2-ethoxy-1,2-dihydroquinoline, N-(isobutoxycarbonyl)-2-isobutoxy-1,2-dihydroquinoline and the like; and disulfide linkages which include oxidizing agents such as iodide, hydrogen peroxide, bis-1,10-phenanthroline complex of cupric ion, and the like.

It is presently preferred to employ a carbodiimide such as 1-ethyl-3-(3-dimethylaminopropyl)-carbodiimide ("EDAC" herein).

Among other analytes, the labile enzymes which may be stabilized in accordance with the invention include, without limitation, oxidoreductases such as dehydrogenases and oxidases, peroxidases, oxygenases and the like; transferases such as methyltransferases, transaldolases and transketolases, acyltransferases, glycosyltransferases, alkyltransferases, transaminases, transphosphorylases, sulphotransferases and the like; hydrolases such as esterases, thiolesterases, phosphoesterases, glycosidases peptidases pyrophosphatases and the like; lyases such as decarboxylases, oxo-acid-lyases and the like; isomerases such as racemases and epimerases, intramolecular oxidoreductases, intramolecular transferases and the like and ligases such as aminoacyl-tRNA synthetases, carboxylases and the like. Mixture of enzymes may also be stabilized.

The biostabilizer and condensing agent are dissolved in water or a water soluble solvent such as dimethyl sulfoxide (DMSO). Dimethyl sulfoxide is the preferred water soluble solvent.

At least one of the solutions used to form the stabilized analyte is aqueous based and may be buffered normally with a zwiterionic buffer preferably 2-amino-2-hydroxymethyl-1,3-propanediol (TRIS) normally used in a concentration of about 0.02 to about 0.15% by weight.

Mixtures of biostabilizers and/or condensing agents may be used.

The analyte solution normally contain the analyte in a concentration of about 0.1 to about 800 mg/ml.

The biostabilizer solution normally contains the biostabilizer in a concentration of from about 0.1 to about 100 mg/ml.

The condensing agent solution normally contains the condensing agent in a concentration of about 0.1 to about 100 mg/ml.

The relative ratios of analyte solution, biostabilizer solution and condensing agent are produced to give a ration of analyte to biostabilizer of 100:1 or less and a ratio as to each other with range from about 100:1:1 to 1:100:5 w/w/w.

### Example 1

(GPO) was chosen as a model to illustrate the invention. Pilots were compounded as shown in Table 1.

**Table 1**

| Pilot # | GPO (10/mg/ml) | EDAC (100 mg/ml) | PSL (20 mg/ml) |
|---|---|---|---|
| 1 | 0.5 ml | 0.18 ml | 0.08 ml |
| 2 | 0.5 ml | 0.05 ml | 0.08 ml |
| 3 | 0.5 ml | 0.18 ml | 0.16 ml |
| 4 | 0.5 ml | 0.10 ml | 0.04 ml |

In preparing the pilots GPO was dissolved in base comprising of 50 mg/ml BSA and 10 mg/ml Tris with no pH adjustment. EDAC and polysuccinylated lysine (PSL) were dissolved in DMSO. The PSL solution was added to GPO dropwise slowly with stirring over an ice bath. The EDAC solution was added to above mixture slowly with stirring at 4°C and the mixture above was stored for 4 days before use. The results as to appearance were:

| Pilot | Appearance |
|---|---|
| 1 | solidified |
| 2 | homogeneous liquid |
| 3 | solidified |
| 4 | homogeneous liquid |

Solidification was likely due to the higher EDAC concentration. The yield for both pilot 2 and 4 is roughly 25 to 30% of theoretical.

Attached FIG. 1 compares the stability of pilots 2 and 4 used in a single reagent triglyceride system with and without the coenzyme FAD as compared to a two component triglyceride reagent with and without FAD. The graph show significant stabilization was achieved, especially with FAD.

In particular, the modified GPO pilots 2 and 4 are inherently more heat stable when compared to the unmodified GPO. Heat stability of both GPO pilots are tremendously enhanced by the presence of 0.02% FAD.

A triglyceride single reagent (Trig Sr) is feasible because the minimal amount of GPO for reagent performance is 400 U/L or 16% of the initial 2500U/L added; test shows there was in excess of 16% recovery of GPO after 3 days at 41°C.

The only ingredient which is deficient after three days at 41°C is GPO. (i.e. 3 days stressed at 41°C reagent is not functional; however, after spiking with GPO, it became totally functional.

### Examples 2 and Control 2

### Stabilization of Glycerol Phosphate Oxidase (GPO).

There was formed a solution of GPO at a concentration of 50 mg/ml by dissolving the GPO in an aqueous base comprising 50mg/ml of bovine serum albumin (BSA) and 10 mg/ml Tris with no pH adjustment.

There was separately formed a solution of EDAC in DMSO in a concentration of 100 mg/ml and a solution of polysuccinylated lysine (PSL) (molecular weight 220,000) at a concentration of 20mg/ml in DMSO.

A PSL solution was added to the GPO solution in proportion of 0.04 ml of PSL solution to 0.5 ml of GPO solution with stirring at a temperature between 0°C and about 10°C.

The EDAC solution was then added in a concentration of 0.1 ml EDAC solution to the mixture of PSL solution and GPO solution over an ice bath at temperature between 0°C and about 10°C. The mixture was stored at 4°C for 4 days and was evaluated for percent active GPO remaining as is shown in Table 2.

**Table 2**

| | 1 day at 4 and 41°C % GPO | | 3 days at 4 and 41°C % GPO | |
|---|---|---|---|---|
| | 4°C | 41°C | 4°C | 41°C |
| Example 2 | 100% | 70% | 100% | 50% |
| Control 2 | 100% | 0% | 100% | 0% |

Table 2 makes clear that the product in Example 2 remains active while the unmodified GPO (Control 2) lost all stability.

### Example 3 and Control 3

### Stabilization of Phosphoenolpyruvate Carboxylase (PEPC).

There was formed a solution of 20 mg/ml of PEPC by dissolving PEPC in an aqueous base containing 50 mg/ml BSA, 10 mg/ml Tris and 50 mg MgOAc without pH adjustment.

Solutions of EDAC and PSL in DMSO at respective concentrations of 100mg/ml and 20mg/ml were formed as in Example 3.

The PSL solution was added to the PEPC solution dropwise with stirring over an ice bath followed by the addition of the EDAC solution, mixture was stored at 4°C for 4 days and incubated at 2 days at 35°C.

Table 3 compares the stability of the PEPC in a carbon dioxide single reagent solution to the unstabilized PEPC of Control 3.

**Table 3**

| Performance of Stabilized PEPC in CO2 Sr | | | | |
|---|---|---|---|---|
| | 1 day at 4 and 41°C % PEPC of 4°C | | 3 days at 4 and 41°C %PEPC of 4°C | |
| | 4°C | 41°C | 4°C | 41°C |
| Example 3 | 100% | 95% | 100% | 90% |
| Control 3 | 100% | 0% | 100% | 0% |

### Example 4 and Control 4

As in Example 2 Horseradish Peroxidase (HRP) was dissolved in an aqueous base containing 50 mg/ml BSA and 10 mg/ml Tris with no pH adjustment. HRP concentration was 30 mg/ml.

EDAC and PSL solutions were formed as in Example 2 as in relative concentrations of 100 mg/ml and 20 mg/ml. The PSL solution was added in an amount of 0.14 ml per 0.5ml of HRP solution dropwise over an ice bath. This was followed by the addition of the EDAC solution in an amount of 0.05 ml per 0.5 ml of HRP solution. The mixture was stored at 4°C for 4 days and evaluated for the stability of HRP in a uric acid single reagent solution.

This is reported in Table 4 where the Control 4 is the unmodified horseradish peroxidase.

**Table 4**

| Performance of Stabilized HRP in Uric Acid SR Model | | | | |
|---|---|---|---|---|
| | 1 day at 4 and 41°C %HRP of 4°C | | 3 days at 4 and 41°C %HRP of 4°C | |
| | 4°C | 41°C | 4°C | 41°C |
| Example 4 | 100% | 70% | 100% | 35% |
| Control 4 | 100% | 0% | 100% | 0% |

### Example 5 and Control 5

### Stabilization of Creatine Kinase (CK).

To 10 ml of water there was added 100 mg of ADP Lithium Salt, 100 mg Tris forming a solution with an adjusted pH of 9.0. Creatine Kinase was dissolved in the solution to a concentration of 50 mg/ml to which there was added for each milliliter of CK solution to 0.4 ml of an PSL solution at a concentration of 20 mg/ml in DMSO dropwise at 4°C, followed by 0.08 ml of a solution of EDAC at a concentration of 100mg/ml in DMSO.

**Table 5**

| Performance of Modified CK in Chemistry Control | | |
|---|---|---|
| | 3 days at 4 and 41°C % of 4°C CK Activity | |
| | 4°C | 41°C |
| Example 5 | 100% | 110% |
| Control 5 | 100% | 30% |

### Example 6 and Control 6

### Glutamate pyruvate transaminase (PT) Stabilization Process.

There is compounding a base containing 5.5ml Plasma Diagnostic Base purchased from Biocell (PDB), 1.4 ml cholesterol concentrate, 3.1 ml water, 0.1 ml Triton X-405, 40 mg of gelatin, 100 mg of Tris base and 0.1 ml of a pyroxidal-5-phosphate (P-5-P) solution at a concentration of 20mg/ml.

PT was added to the base at a concentration of 5 mg/ml to each milliliter of the base there was added 0.04 ml of PSL in a concentration of 20 mg/ml in DMSO dropwise at 4°C followed by a dropwise addition of 0.25 ml of EDAC at a concentration of 100 mg/ml in DMSO. The combined solutions were maintain at 4°C for 2 days and the performance as a chemistry control is shown in Table 5.

**Table 6**

| Performance of Modified PT in Chemistry Control | | |
|---|---|---|
| | 6 days at 4 and 41°C % of 4°C PT Activity | |
| | 4°C | 41°C |
| Example 6 | 100% | 95% |
| Control 6 | 100% | 0% |

Labile Enzymes are proteins with very specific 3 dimensional conformations which are extremely crucial to the specific catalytic functions in aqueous solutions. The useful conformation of the enzymes may be destroyed by microbial action, oxidation, self folding or unfolding, aggregation with other nonspecific proteins, and aggregation with other similar proteins.

The instant invention of enzyme stabilization is based upon the caging of the labile enzyme molecules in their catalytically active conformations, through a mild condensing agent, with non-reactive soluble biostabilizers especially amino acid polymers. This process creates a physical barrier between the above identified deleterious effects and the useful enzyme conformations.

The condensing agent, such as 1-ethyl-3-(3-Dimethylaminoproply)-Carbodiimide or EDAC, has the function depicted in FIG. 2. FIG. 2, depicts how a carboxy functional biostabilizer P₁ reacts with the condensing agent EDAC which in turn cross-links with the enzyme to be stabilized, P₂ to form a product in which the enzyme P₂ is coupled through EDAC to the biostabilizer in its useful enzyme configuration.

## Claims

1. A stable water soluble analyte which comprises a water soluble reaction product of a labile analyte with a water soluble biostabilizer in an aqueous media in the presence of a condensing agent; said condensing agent causing or entering into the reaction between the labile analyte and biostabilizer.

2. A stable analyte as claimed in claim 1 in which the biostabilizer is a hydrophilic molecule of defined molecular weight which contain sufficient functional groups to stabilize the labile analyte and in which the weight ratio of the labile analyte to biostabilizer is about 100:1 or less.

3. A stable analyte as claimed in claims 1 or 2 in which the biostabilizer comprises the biopolymers and proteins.

4. A stable analyte as claimed in any one of claims 1 to 3 in which the biopolymer comprises a compound the selected from the group consisting of polyamino acids, polysaccharides and mixtures thereof.

5. A stable analyte as claimed in claim 4 in which the polyamino acid comprises a polyamino acid selection from the group consisting of poly-arginine, poly-dl-lysine, poly-l-lysine, poly-dl-aspartate, poly-l-aspartate, poly-d-glutamic acid, and polysuccinylated-lysine and mixtures thereof.

6. A stable analyte as claimed in claim 5 in which the biostabilizer is a polysuccinylated-lysine having a molecular weight of about 220,000.

7. A stable analyte as claimed in claims 1 to 3 in which the biostabilizer comprises a protein selected from the group consisting of naturally occurring proteins and modified proteins.

8. A stable analyte as claimed in claim 7 in which the modified protein contains sufficient functional group to stabilize the labile analyte.

9. A stable analyte as claimed in any one of claims 1 to 9 in which the functional group comprises functional groups selected from the group comprising N-terminal amino groups, C-terminal carboxyl groups, thiol groups, thioether groups, imidazole groups, guanidino groups, phenolic hydroxyl groups, indolyl groups and mixtures thereof.

10. A stable analyte as claimed in any one of claims 1 to 3 in which the biostabilizer comprises a protein selected from the group consisting essentially of gelatin, albumin, collagen, fibrinogen and mixtures thereof.

11. A stable analyte as claimed in any one of the previous claims in which the condensing agent is a reagent selected from the group consisting of homobifunctional, heterobifunctional, zero-length reagents and mixtures thereof.

12. A stable analyte as claimed in any one of the previous claims in which the condensing agent comprises a reagent capable of reacting with a functional group selected from the group consisting of N-terminal amino groups, C-terminal carboxyl groups, thiol groups, thioether groups, imidazole groups, guanidino groups, phenolic hydroxyl groups, indolyl groups and mixtures thereof.

13. A stable analyte as claimed in any one of the previous claims in which the condensing agent is a carbodiimide.

14. A stable analyte as claimed in claim 13 in which the condensing agent is 1-ethyl-3-(3-dimethylaminopropyl)-carbodiimide.

15. A stable analyte as claimed in any one of the previous claims in which the labile analytes is an enzyme.

16. A stable analyte formed by reaction of a labile enzyme selected from the group consisting of glycerol phosphate oxidase, phosphoenolpyruvate carboxylase, horseradish peroxidase, creatine kinase, uricase, and alanine aminotransferase reacted with polysuccinylated-lysine in the presence of 1-ethyl-3-(3-dimethylaminopropyl) carbodiimide as a condensing or linking agent.

17. A process for stabilizing labile analytes which comprises:
a) slowly adding to a continuously agitated aqueous solution of the labile analyte at a temperature of about greater than 0° and up to about 10°C a solution of biostabilizer while maintaining the temperature of about greater than 0°and up to about 10°C;
b) adding to the solution of the labile analyte and biostabilizer a condensing or linking agent while maintaining the mixture at a temperature of about greater than 0° and up to about 10°C to form a net aqueous mixture which the weight ratio of labile analyte to biostabilizer is about 100:1 or less;
c) allowing the net aqueous mixture to undergo a stabilization reaction at a temperature of from greater than 0° and up to about 10°C to form an analyte stabilized by the biostabilizer or biostabilizer and condensing or linking agent.

18. A process as claimed in claim 17 in which the stabilizing reaction is accelerated by raising the temperature to at least about 35°C.

19. A process as claimed in claims 17 or 18 in which the labile analyte is an enzyme, the biostabilizer is a hydrophilic molecule of defined molecular weight which contains sufficient functional groups to stabilize the labile analyte and the condensing agent comprises a reagent capable of reacting with the functional group selected from the group consisting of N-terminal amino groups, C-terminal carboxyl groups, thiol groups, thioether groups, imidazole groups, guanidino groups, phenolic hydroxyl groups, and indolyl groups and mixtures thereof.

20. A process as claimed in any one of claims 17 to 19 in which the labile analyte is an enzyme, the biostabilizer is a polysuccinylated-lysine and the condensing agent is 1-ethyl-3-(3-dimethylaminopropyl)carbodiimide.
